# FASCICULE DE BREVET EUROPEEN

(11) **EP 0 528 708 B1**
(45) Date de publication et mention de la délivrance du brevet: **17.04.1996**
(21) Numéro de dépôt: 92402101.7
(22) Date de dépôt: 21.07.1992
(51) Int. Cl.: G01N 33/546, G01N 33/543, G01N 15/04, B03C 1/28

(54) **Procédé et dispositif magnétique d'analyse immunologique sur phase solide**
Verfahren und magnetische Vorrichtung zur immunologischen Analyse von Festphasen
Method and magnetic device for solid phase immunological assay

(30) Priorité: 22.07.1991 FR 9109242
(43) Date de publication de la demande: 24.02.1993
(73) Titulaire: PASTEUR SANOFI DIAGNOSTICS, F-92430 Marnes La Coquette (FR)
(72) Inventeur: Matte, Claude, F-75005 Paris (FR); Muller, Anne, F-91190 Gif/Yvette (FR)
(74) Mandataire: Le Guen, Gérard

(56) Documents cités:
- EP-A- 0 262 760
- EP-A- 0 351 857
- EP-A- 0 417 301
- WO-A-87/05536

## Description

La présente invention concerne un procédé de dosage ou de détection immunologique de substances biologiques, notamment d'antigènes ou d'anticorps, et des dispositifs pour la mise en oeuvre de ce procédé.

De nombreuses techniques, plus ou moins sensibles et spécifiques, permettent de déceler la présence d'antigènes ou celle d'anticorps dans les milieux biologiques grâce à la formation de liaisons de type immunologique entre la substance à doser et une ou des substances connues.

Certaines mettent en jeu un phénomène d'immunoadhérence, décrit notamment dans Amer. J. of Clinical Pathology 82 (6) - 719-721 (1984). Dans cette méthode, l'échantillon à étudier est introduit dans un récipient, généralement la cupule d'une plaque de microtitration, dont les parois portent des molécules ou des cellules ayant une affinité spécifique pour la substance à doser; une suspension de particules sensibilisées, c'est-à-dire qui portent une substance susceptible de se lier par affinité à la substance à doser, est aussi introduite et la répartition de ces particules après une phase de sédimentation dynamique est étudiée; en présence de la substance recherchée, les particules tapissent régulièrement les parois auxquelles elles sont reliées par l'intermédiaire de liaisons de type immunologique intervenant entre la substance fixée sur la paroi, celle à doser et la substance présente sur la particule : un voile régulier est observable sur le fond de la cupule; en l'absence de la substance recherchée, les particules se réunissent au fond, autour du point de convergence des parois formant une tache centrale plus opaque. Il est évident qu'en l'absence de force extérieure appliquée sur les particules, leur sédimentation serait trop lente pour que le procédé trouve son application en analyse biologique rapide de routine et on sait qu'une centrifugation convenable permet d'acccélérer ce type de sédimentation, mais elle impose l'utilisation d'un matériel spécifique volumineux, de précision et à réglage délicat; en effet, d'une part la force centrifuge appliquée doit être exactement parallèle à l'axe de symétrie des récipients, sous peine d'avoir des profils de répartition différents suivant la place du récipient par rapport à l'axe de la rotation et, d'autre part, les conditions de centrifugation doivent être, pour obtenir une bonne sensibilité et éviter des résultats faux, fixées pour chaque type de substance à doser et réactif puisque la durée et la force centrifuge en dépendent; en outre, ces techniques avec centrifugation, même si elles sont utilisées pour des analyses de routine, ne se prêtent pas à une automatisation compacte, simple, peu coûteuse.

On a récemment proposé, dans la demande de brevet WO 90/09590, ainsi que dans la demande de brevet EP-A-0 351 857, d'accelérer la sédimentation des particules sensibilisées par action d'une force magnétique au lieu d'une force centrifuge, en utilisant des particules sensibilisées contenant un noyau magnétique - procédé qui supprime les inconvénients dûs à l'utilisation d'une centrifugeuse.

Néanmoins la demanderesse a constaté, comme on le verra plus loin, que cette technique, notamment décrite dans WO 90/09590, ne permet pas de différencier nettement les réactions positives faibles des réactions négatives, de telle sorte qu'elle n'est pas suffisamment sensible pour les recherches d'anticorps antiérythrocytaires irréguliers et d'antigènes en contrôle prétransfusionnel, ou d'antigènes lymphocytaires avant les transplantations tissulaires.

La présente invention fournit un procédé nettement plus sensible, spécifique, rapide et en partie ou totalement automatisable puisque l'observation des particules au fond des récipients, qu'ils soient transparents ou opaques, peut être effectuée à l'aide d'un photomètre, tandis que les opérations de remplissage des récipients de réaction, les incubations et les lavages sont réalisables avec des dispositifs classiques, connus de l'homme du métier.

La présente invention a pour objet un procédé de dosage ou de détection par immunoadhérence d'une substance biologique susceptible d'être présente dans un échantillon, qui consiste à introduire l'échantillon dans un récipient dont les parois portent un élément ayant une affinité immunologique spécifique pour la substance à rechercher, à ajouter des particules magnétiques portant une substance ayant une affinité immunologique spécifique pour la substance à rechercher, à les soumettre à plusieurs actions magnétiques successives pour accélérer le dépôt desdites particules sur les parois et déplacer celles qui ne sont pas entrées dans des liaisons spécifiques avec la substance à rechercher qui adhère aux parois par l'intermédiaire de l'élément affine qui y est fixé, et à observer les particules déposées.

Dans un premier mode de réalisation, le procédé selon l'invention consiste :
a) à introduire ledit échantillon dans un récipient qui porte sur ses parois un élément ayant une affinité immunologique spécifique pour la substance à rechercher,
b) à ajouter des particules magnétiques portant une substance ayant une affinité immunologique spécifique pour la substance à rechercher,
c) à soumettre les particules à l'action d'un champ magnétique statique pour plaquer les particules sur les parois, et permettre ainsi la réaction immunologique,
d) puis à soumettre les particules à l'action d'un champ magnétique d'orientation variable pour arracher et réunir au fond les particules qui ne sont pas fixées par liaison spécifique aux parois par l'intermédiaire de la substance à rechercher et de l'élément affine solidaire de la paroi,
   et enfin,
e) à observer les particules déposées.

Selon une variante de ce premier mode de réalisation, on soumet en outre les particules, avant l'observation finale, à un champ magnétique statique pour retirer du récipient les particules qui n'y adhèrent pas par l'intermédiaire de liaisons immunologiques. En pratique, on peut introduire dans le récipient une tige convenablement aimantée sur laquelle viennent adhérer uniquement les particules qui n'adhèrent pas spécifiquement aux parois du récipient.

Dans un second mode de réalisation, le procédé selon l'invention consiste :
a) à introduire ledit échantillon dans un récipient qui porte sur ses parois un élément ayant une affinité immunologique spécifique pour la substance à rechercher,
b) à ajouter des particules magnétiques portant une substance ayant une affinité immunologique pour ladite substance à rechercher,
c) à soumettre les particules à l'action d'un champ magnétique statique pour plaquer les particules sur les parois, et permettre ainsi la réaction immunologique caractéristique,
d) puis à soumettre le récipient à l'action d'un champ magnétique statique, notamment à l'aide d'une tige convenablement aimantée, pour retirer du récipient les particules qui n'adhèrent pas à ses parois par une liaison de type immunologique,
e) et enfin à observer les particules déposées.

De façon connue, on laisse, en général, incuber ledit échantillon à étudier dans le récipient sensibilisé pendant un certain temps, par exemple jusqu'à 30 minutes, à une température comprise entre environ 20° C et 45° C avant d'ajouter les particules magnétiques.

En outre on effectue, en général, une étape de lavage entre ces deux premières étapes du procédé de l'invention, en particulier lorsque les particules magnétiques sont sensibilisées avec une substance qui n'aurait pas d'affinité uniquement pour la substance à rechercher.

En général, les dosages immunologiques de routine sont effectués en parallèle dans des plaques de microtitration qui comportent des séries de godets aussi appelés cupules ou puits, parfois jusqu'à une centaine, d'une capacité de 350 µl environ, disposés en rangées parallèles ou dans des godets alignés sur des barrettes supports, mais tout autre récipient de dimension convenable pourraît être utilisé.

La présente invention a également pour objet des dispositifs pour la mise en oeuvre du procédé selon l'invention.

Dans ce qui suit, on décrit les dispositifs selon l'invention en se référant à des plaques de microtitration, mais il est entendu que l'invention n'est pas limitée à ce type de récipients, ni à un appareil comportant une seule plaque. Dans ces plaques, les cupules peuvent être à fond plat, conique, tronconique ou hémisphérique.

Un dispositif selon l'invention, utile pour la mise en oeuvre du premier mode de réalisation du procédé, comprend :
- un support pour maintenir une plaque de microtitration horizontale, et susceptible de donner à l'ensemble de la plaque un mouvement de translation circulaire horizontal dont l'amplitude est comprise entre 1 mm et 15 mm de rayon, en fonction de la dimension des cupules ;
- en-dessous, un ensemble d'aimants dont les pôles supérieurs sont parallèles à la plaque et sont soit disposés sous chaque cupule en damier éventuellement alternativement Nord et Sud, l'espace entre eux étant de l'ordre de 2 mm au moins, soit avantageusement disposés en bande sous chaque rangée de cupules, ceux de deux bandes voisines étant alternativement Nord et Sud, et l'espace entre deux bandes étant très faible, de l'ordre d' 1/10 mm ou moins, les bandes pouvant même être jointives.

Par translation circulaire, on entend un mouvement au cours duquel le centre du récipient décrit une trajectoire circulaire mais n'effectue aucune rotation autour de son axe.

Selon une alternative de réalisation, le dispositif peut comprendre un support de plaque fixe, et un ensemble d'aimants susceptible d'effectuer un mouvement de translation circulaire horizontal.

Un autre dispositif selon l'invention, utile pour la mise en oeuvre de la variante du premier mode de réalisation ou pour la mise en oeuvre du second mode de réalisation comprend en outre une série de tiges cylindriques aimantées, de diamètre inférieur à celui des cupules, et des moyens pour les introduire simultanément dans toutes les cupules, le long de leur axe, jusqu'à une faible distance du fond, par exemple entre 0,1 et 1,5 mm et les en extraire.

Ces tiges peuvent être en fer doux ou en acier, traitées pour les rendre inattaquables par le milieu réactionnel; elles sont alors pendant leur emploi en contact magnétique avec un aimant permanent, qui les magnétise. On peut avoir un seul aimant pour toutes les tiges, ou un petit aimant pour chacune. Par ailleurs, chaque tige peut être elle-même un aimant permanent. La forme de l'extrémité inférieure des tiges est, de préférence, adaptée à celle du récipient dans lequel elles sont plongées, hémisphérique, conique ou plate selon le cas, mais on peut aussi, notamment, utiliser une extrémité conique dans une cupule hémisphérique.

Les tiges dont la longueur peut être supérieure à la profondeur des cupules et qui peuvent être plus larges au sommet sont supportées par une grille ou une plaque ainsi qu'éventuellement les aimants permanents.

Pour la mise en oeuvre du deuxième mode de réalisation du procédé, l'ensemble d'aimants alternés du premier dispositif peut être remplacé par tout moyen permettant de créer dans la zone où se trouvent les récipients un champ magnétique, qui attirera les particules vers les parois, tel qu'une plaque uniformément aimantée; en outre, dans ce cas, le support de plaque de microtitration et les aimants sont immobiles l'un par rapport à l'autre.

Les récipients pourront être remplis et lavés manuellement ou à l'aide de moyens mécaniques connus dans des postes appropriés et on pourra associer aux moyens de traitement magnétique, selon l'invention, des moyens classiques de remplissage et de lavage des plaques de microtitration.

En outre, l'observation des profils de répartition des particules pourra être effectuée à l'oeil nu, de préférence à l'aide d'un dispositif grossissant, tel qu'une loupe binoculaire et pour les très grandes sensibilités avec un photomètre convenable couplé à un système d'enregistrement; on utilise dans ce dernier cas, comme il est connu, une plaque de microtitration transparente.

Dans le procédé selon l'invention, la substance à rechercher peut être un antigène libre ou lié à la surface d'une cellule telle que virus, lymphocyte, plaquette, érythrocyte, ou un anticorps, dirigé contre un antigène cellulaire ou tissulaire ou contre un allergène, présent dans le sérum ou dans un autre milieu biologique.

Les substances ayant une affinité pour la substance à rechercher, c'est-à-dire susceptibles de former des liaisons de type immunologique avec elle, sont alors respectivement des anticorps ou des antigènes. L'élément de capture, immobilisé sur les parois du récipient de titration, devra fixer spécifiquement la substance à doser, tandis que la substance révélatrice, portée par les particules magnétiques, pourra ne pas être spécifique. On connaît de nombreuses substances affines appropriées, couramment utilisées dans les analyses immunologiques, comme les anticorps polyclonaux ou monoclonaux, les antiglobulines, les lectines, les globules rouges, les protéines virales et l'homme du métier choisira celle convenant à la détermination souhaitée, sans difficulté.

L'élément fixé sur les parois peut être un anticorps ou un antigène, libres ou intégrés dans la membrane d'une cellule entière, d'un stroma ou provenant d'un lysat cellulaire.

L'élément de capture est fixé sur les parois des cupules par des techniques bien connues du spécialiste, que ce soit par adsorption, lorsque les cupules sont en polystyrène, en chlorure de polyvinyle ou en polyméthacrylate, par liaison chimique covalente avec un composé mélangé au matériau de la plaque ou déposé sur les parois des cupules dans un film adhérent ou par tout autre moyen connu du spécialiste comme celui mentionné dans EP-A-0350233 selon lequel des globules rouges sont liés par l'intermédiaire de colorants ioniques.

On trouve dans le commerce un certain nombre de plaques de microtitration, dites sensibilisées, dont les cupules portent l'élément de capture ou sont prêtes à être sensibilisées par des techniques connues. Le spécialiste sait que toutes les plaques ne conviendront pas pour les dosages du type de ceux de l'invention, par immunoadhérence; il est évidemment souhaitable, pour qu'il n'y ait pas de faux positifs ou de faux négatifs, que la densité des sites de liaison soit régulière et telle que les particules révélatrices se disposent sur les parois en une seule couche, de préférence pratiquement continue.

Les particules magnétiques seront de petite taille, de l'ordre de 0,5 µm à 5 µm et de préférence entre 0,8 µm et 1,7 µm et de granulométrie assez homogène, en général, constituées d'un polymère naturel ou synthétique, qui n'adhère pas spontanément fortement au matériau des godets; le polymère peut enrober un noyau magnétique ou des éléments magnétiques tels que fer, ferrites et alliages qui sont dispersés dans la masse polymérique. Des particules convenables sont commercialisées, et on peut citer celles de marque Dynabeads^{R} de la société Dynal ou celles de marque Estapor^{R} de la société Rhone-Poulenc, qui sont composées d'un latex de polystyrène, pouvant être chargé, à 40 % en poids, en ferrite. Des particules colorées peuvent aussi être utilisées.

La substance portée par les particules magnétiques doit avoir une affinité pour la substance à doser; ainsi une antiimmunoglobuline spécifique d'espèce pour le dosage d'un anticorps ou un anticorps polyclonal, ou monoclonal pour le dosage d'un antigène, conviennent. Ils seront fixés sur la particule par une des techniques connues de l'homme du métier, similaires à celles utilisées pour la fixation dans les cupules; plusieurs molécules sont en général fixées simultanément sur une même particule, ce qui permet, si la densité de répartition des substances à doser immobilisées sur les parois est convenable, d'avoir une excellente adhérence de la particule.

Dans la première phase du traitement magnétique, les cupules de titration sont placées dans des champs magnétiques statiques, dont la répartition et l'intensité sont identiques pour toutes les cupules, dirigés de telle sorte que les particules soient entraînées vers le bas et se déposent régulièrement sur la surface des parois. La force magnétique doit être suffisamment intense pour que la sédimentation soit rapide, sans être excessive pour éviter que toutes les particules se réunissent au point le plus bas, après avoir été arrachées des parois où elles s'étaient préalablement déposées.

Dans cette phase, lorsque les particules arrivent près des parois sensibilisées, des liaisons d'affinité peuvent se créer entre la substance révélatrice portée par la particule et la substance à doser, immobilisée sur la paroi par l'intermédiaire de l'élément de capture spécifique.

Dans les modes de réalisation du procédé où les particules sont soumises à l'action d'un champ magnétique d'orientation variable, au cours de cette phase les particules faiblement ou non liées sont arrachées et glissent le long des parois pour se réunir dans la zone la plus basse des cupules.

Un moyen préféré, selon l'invention, pour faire varier cette orientation, consiste, comme mentionné précédemment, à soumettre la plaque de microtitration à des mouvements de translation circulaire de faible diamètre au-dessus de l'ensemble d'aimants de telle sorte que l'axe d'une cupule parcourt un cercle dont le centre est situé à l'intersection de la ligne joignant son axe à celui d'une cupule contigue, située au-dessus d'un aimant de sens opposé à celui au-dessus duquel elle est placée, et de la ligne d'entrefer de ces deux aimants; le rayon dudit cercle est inférieur à la demi-largeur d'un aimant, de préférence entre 10 et 40 % de cette largeur.

Dans ces conditions, tous les points du fond de chaque récipient se trouvent à un moment au-dessus de la ligne d'entrefer, où le champ magnétique est maximum, ou à son voisinage, de telle sorte que toutes les particules faiblement liées sont arrachées des parois en très peu de temps, en moins de 5 minutes et mieux dans la plupart des cas en environ 2 minutes.

L'intensité des champs magnétiques à appliquer dépend notamment de la taille, de la densité des particules et de leur concentration, de la viscosité des milieux réactionnels, de l'intensité des forces de liaison d'affinité, de la géométrie des cupules, et le spécialiste déterminera par des essais préalables, leur valeur ainsi que les durées convenables des différentes phases de traitement magnétique qui en dépendent, pour obtenir une sensibilité optimale et l'absence de faux positifs et négatifs pour une durée de traitement raisonnable.

De préférence, le fond des cupules sera placé à une faible distance au-dessus de l'ensemble d'aimants, ce qui permettra l'utilisation d'aimants de faible puissance, notamment d'aimants constitués par des bandes de polymères magnétisés par inclusion de particules aimantées, que l'on trouve dans le commerce; cette distance sera, en général, de l'ordre du mm, de 0,1 à 5 mm, pour des inductions magnétiques inférieures à 0,5 tessla.

Pour les recherches d'antigènes ou d'anticorps dans des plaques de microtitration courantes, de 12,8 cm x 8,6 cm comportant 96 cupules disposées en 12 x 8 rangées parallèles avec des particules magnétiques de diamètre compris entre 0,5 µm et 5 µm et de densité inférieure ou égale à 2 environ, les intensités moyennes d'induction magnétique sous le fond des cupules sont de 0,2 à 0,4 tessla, les tiges plongeantes ont un diamètre de 3 mm, une longueur de 10 mm au moins et elles créent au voisinage de leur pièce polaire une induction magnétique de 0,10 à 0,20 tessla. La durée de la première phase de dépôt des particules dans les champs fixes est de quelques secondes à 5 minutes, tandis que celle de déplacement dans les champs magnétiques est inférieure à 5 minutes, de l'ordre de 2 minutes environ en général pour une vitesse de rotation comprise entre 0,5 et 4 tours par seconde; enfin, la durée d'action des tiges aimantées sera en général de 5 secondes à quelques minutes, éventuellement en plusieurs plongées successives.

Le procédé de l'invention, dans ses différentes variantes, peut être effectué manuellement, avec un appareillage simplifié de traitement magnétique, mais il peut aussi être effectué dans un appareil automatique d'analyse biologique qui comprend, outre les éléments classiquement présents dans ce type d'appareil effectuant des dosages immunologiques, au moins un dispositif de traitement magnétique selon la présente invention.

Ainsi, un automate susceptible de réaliser la recherche d'antigènes'ou d'anticorps par le procédé d'immunoadhérence de l'invention comprend un passeur d'échantillons, un poste de remplissage des cupules de plaque de microtitration avec les échantillons et les réactifs dont les particules magnétiques en suspension, un poste d'incubation, un poste de lavage des cupules, ainsi qu'un poste permettant la réalisation des traitements magnétiques selon l'invention et un poste de lecture photométrique permettant l'observation, quantitative ou non, des particules déposées dans les cupules, les différents postes étant sous le contrôle d'un micro-ordinateur dirigeant les différentes étapes du dosage, et son interprétation.

Afin de mieux faire comprendre l'invention, des modes de réalisation particuliers vont être décrits en se référant aux Figures annexées sur lesquelles :
- la Figure 1 représente un dispositif selon l'invention en vue de dessus, avec arrachage partiel,
- la Figure 2 représente, en coupe selon 2-2 et en vue agrandie, une partie du dispositif montrant la plaque de microtitration et les aimants, au cours du traitement magnétique statique initial,
- la Figure 3 représente, en coupe selon 3-3, le dispositif selon la Figure 1,
- la Figure 4 représente, en coupe et en vue agrandie, une partie d'un dispositif utilisé pour la mise en oeuvre d'une variante.

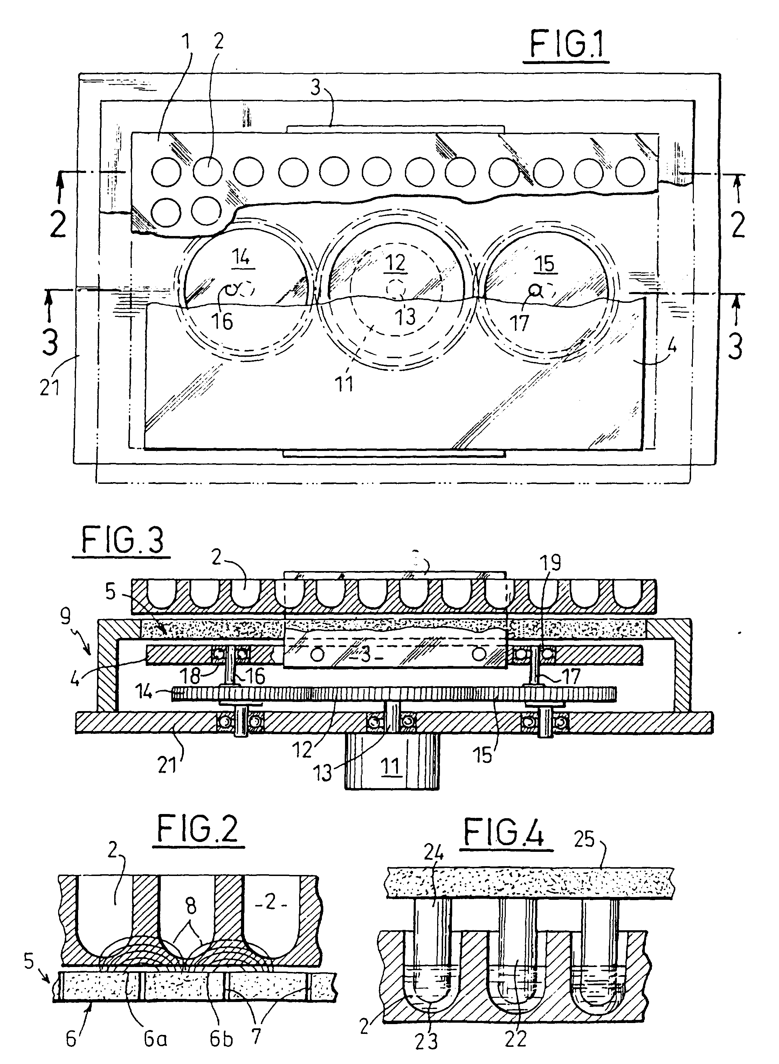

On a représenté sur les Figures 1 à 3 un dispositif pour la mise en oeuvre du procédé selon l'invention conformément à son premier mode de réalisation, comprenant des moyens de traitement magnétique pour la réalisation de dosages par immunoadhérence à l'aide de plaques de microtitration.

Le dispositif représenté sur les Figures 1 à 3 comprend une plaque de microtitration 1 comprenant un ensemble de cupules 2. Cette plaque est fixée horizontalement sur les deux parties d'un support 3, lui-même fixé sur une plaque 4 disposée horizontalement qui sera décrite plus en détail plus loin en référence à la Figure 3.

Comme représenté sur la Figure 2, en-dessous de la plaque de microtitration 1, à faible distance et parallèlement à cette plaque, se trouve un ensemble 5 d'aimants 6 en forme de bandes disposées sous chaque rangée de cupules 2 et dont les pôles supérieurs sont alternativement Nord 6a et Sud 6b, un très faible espace 7 (entrefer) étant ménagé entre deux bandes.

Le champ magnétique statique régnant au-dessus des aimants 6 est représenté symboliquement en 8.

L'ensemble 5 d'aimants 6 est solidaire d'un châssis 9 parallélépipédique sous lequel est fixé un moteur 11.

Le moteur 11 entraîne un pignon central 12 par un axe 13. Le pignon moteur engrène les pignons latéraux 14, 15, lesquels sont chacun porteur d'un petit axe excentré 16, 17 servant de moyeu aux roulements à billes 18, 19.

Ces roulements sont sertis dans la plaque 4 qui sera donc animée d'un mouvement de translation circulaire lors de la rotation des pignons porteurs des petits axes excentrés 16, 17.

Les pignons 12, 14, 15 sont montés sur la plaque inférieure 21 du châssis 9.

Pour l'application du champ magnétique statique la disposition des aimants telle que représentée sur la Fig. 1 est utilisée. Le champ magnétique statique permet de plaquer les particules qui sont introduites dans les cupules 2 contre les parois.

Pour l'application du champ magnétique d'orientation variable, le moteur 11 est mis en rotation et la plaque de titration 1 solidaire de la plaque 4, est soumise à des mouvements de translation circulaire.

Ces mouvements de translation circulaire des cupules 2 au-dessus des champs magnétiques permettent d'obtenir au sein des cupules 2, non seulement un champ magnétique d'orientation variable, mais également une légère agitation du contenu des cupules 2 qui contribue à la séparation des particules non liées ou faiblement liées.

De préférence, avant d'effectuer le mouvement de translation circulaire, la plaque est déplacée par rapport au système d'aimants, parallèlement à une rangée de cupules, dans la direction d'alternance des pôles d'aimants 6a et 6b, sur une longueur égale à environ la demi-distance entre les axes de cupules; par ce moyen, l'axe de chaque récipient se trouvera pendant les rotations plus proche de la zone des entrefers 7, où le champ magnétique est maximal. Le support est alors muni d'un moyen permettant cette courte translation.

Dans un mode de réalisation particulièrement préféré, l'axe de chaque récipient est positionné d'emblée au-dessus de l'entrefer 7.

On a représenté sur la Figure 4 un dispositif utile pour la mise en oeuvre de la variante du premier mode de réalisation du procédé ou pour la mise en oeuvre du deuxième mode de réalisation du procédé.

Le dispositif représenté sur la Figure 4 comprend un ensemble de tiges aimantées 22 disposées suivant le même arrangement que les cupules 2. Chaque tige 22 est en fer doux ou acier; elle est de forme cylindrique; elle possède une extrémité inférieure 23 hémisphérique et une extrémité supérieure 24 au contact d'un aimant permanent 25.

Ce dispositif est destiné à être introduit dans les cupules 2 de la plaque de microtitration après que cette plaque ait été soumise à un champ magnétique statique et/ou tournant, et alors que chaque cupule est encore remplie de liquide de la réaction. Les tiges aimantées 22 sont abaissées à l'aide de moyens non représentés et viennent plonger dans les cupules 2 jusqu'à ce que les extrémités inférieures 23 des tiges soient à une courte distance du fond des cupules. Le réglage de la distance et le centrage des tiges 22 sont assurés par des moyens classiques non représentés.

Les tiges aimantées exercent une force d'attraction dans un sens qui est sensiblement inverse de celui du premier champ magnétique (statique) sur les particules déposées mais seules celles non fixées ou faiblement fixées par des liaisons immunologiques sur les parois des cupules 2 seront extraites.

Dans ce qui suit, on décrit des exemples de mise en oeuvre du procédé selon l'invention, en utilisant les dispositifs de traitement magnétique pour plaque de microtitration précédemment décrits, pour le dépistage d'anticorps anti-érythrocytaires dans le sérum, ainsi qu'à titre comparatif, les résultats obtenus lors d'une sédimentation magnétique sous l'action d'un champ constant, comme décrit dans la demande de brevet WO 90/09590, précédemment citée, ou avec le test commercialisé par la société Immucor Inc - Norcross -U.S.A. sous la marque Capture R^{R} qui implique une centrifugation.

Ce dernier test de dépistage des anticorps irréguliers est effectué par une technique d'immunoadhérence, dans des plaques auxquelles ont été fixées des hématies "tests" comportant les antigènes correspondants aux anticorps à déceler. Ce procédé implique :
- la distribution des échantillons à tester et des témoins dans les godets sensibilisés,
- une incubation assez longue de 20 minutes environ,
- 6 lavages successifs avec une solution isotonique pour éliminer toutes les substances indésirables,
- l'addition d'hématies révélatrices, c'est-à-dire sur lesquelles ont été fixés des anticorps antiimmunoglobulines humaines,
- une centrifugation, dans une centrifugeuse pour plaque à godets, durant une minute à 1000 g,
   puis l'observation des profils de distribution des hématies au fond des cupules; l'ensemble des opérations pour une plaque entière effectuée manuellement demande environ 1 heure.

Le procédé selon l'invention est nettement plus sensible que ces deux techniques connues.

### EXEMPLE 1

Dosage d'un anticorps anti-érythrocytaire irrégulier.

### a) Préparation des particules magnétiques revêtues d'antiglobuline humaine.

Des particules magnétiques de latex, brunes, de marque Estapor^{R} commercialisées par Rhone-Poulenc (granulométrie 0,8 µm) sont lavées avec une solution aqueuse de KOH (pH 9-10) puis mises en suspension à la concentration de 1,2 % (p/V) dans un tampon GBS, composé de glycine 0,1 M et NaCl 0,14 M et ajusté à pH 8,2 par addition de NaOH.

On prépare par ailleurs dans le même tampon une solution d'un anticorps anti-IgG humaines de concentration 0,2 mg/ml.

La solution et la suspension sont mélangées volume à volume et maintenues pendant 45 minutes, sous agitation à 37° C.

Les particules sensibilisées sont alors séparées et lavées abondamment avec le tampon GBS contenant 0,1 % de sérum albumine bovine.

### b) Préparation des solutions d'anticorps à étudier.

On prépare des dilutions, en progression géométrique de raison 2, d'une solution mère à 1 % (p/V) d'un anticorps anti-D, dans une solution aqueuse de NaCl (0,9 % - p/V) contenant de la sérum albumine bovine (6 % - p/V).

### c) Titrage des anticorps.

Dans les essais qui suivent, on utilise une plaque de microtitration en polystyrène de 12,8 cm x 8,6 cm, dont les 96 cupules sont en forme de U, prétraitées pour fixer les globules rouges; cette plaque est commercialisée par Immucor Inc. sous la marque Capture R^{R}.

Une suspension de globules rouges lavés de concentration 0,5 % (p/V) portant l'antigène correspondant à l'anticorps à doser est distribuée dans les cupules; la plaque est centrifugée et les cupules lavées avec du sérum physiologique, puis on y introduit 50 µl de chaque dilution de la solution d'anticorps avec 100 µl de la solution aqueuse de faible force ionique convenable. Dans quelques cupules, on introduit un témoin négatif constitué de sérum humain inerte de groupe AB.

La plaque est incubée 20 minutes à 37°C puis les cupules sont lavées plusieurs fois avec une solution aqueuse de NaCl (0,9 % - p/V). On y introduit alors 50 µl de la suspension de particules magnétiques précédemment préparée amenée à la concentration de 0,02 % en tampon GBS pH 8,2 additionné de 0,1 % de sérum albumine bovine.

La plaque est alors déposée sur le dispositif de traitement magnétique représenté sur les Figures 1 à 3, dont les caractéristiques sont les suivantes : ensemble d'aimants formé de bandes de Ferriflex^{R} (caoutchouc chargé de particules magnétiques) commercialisé par la société DIC (France), d'épaisseurs 5 mm, de largeurs 9 mm et de longueurs 70 mm, situé à moins de 0,8 mm sous le fond des cupules, disposées parallèlement et de telle sorte que les axes des cupules soient alignés sur les milieux des bandes ou mieux sur les entrefers.

La plaque est laissée 2 secondes sur son support, puis le support de la plaque est animé pendant 2 minutes, d'un mouvement de rotation dont le rayon est de 3 mm à la vitesse de 50 t/minutes environ.

On observe alors à l'oeil nu la répartition des particules sur le fond des cupules; pour une réaction négative on observe un gros point brun au milieu du fond de la cupule, tandis que pour une réaction positive on observe un voile brun sur tout le fond.

On différencie encore nettement le témoin négatif, des cupules contenant l'anti-D à la dilution au 1/128^{e} précédemment préparée à partir de la solution mère à 1 %.

A titre de comparaison, on a effectué un essai avec une microplaque sensibilisée de la même façon et avec les mêmes dilutions, mais en utilisant au lieu des particules magnétiques sensibilisées, les globules rouges sensibilisés commercialisés par la société Immucor pour son test Capture-R^{R} ; la phase de traitement magnétique a été remplacée par une centrifugation de la plaque pendant 1 minute à 1000 g, comme préconisé par cette société; on a alors pu différencier les cupules positives et négatives à l'oeil seulement jusqu'à une dilution au 1/32^{e} de la solution mère.

On a aussi préparé comme précédemment les plaques sensibilisées ainsi que des particules magnétiques de 4,5 µm Dynabeads^{R} commercialisées par Dynal sous la référence M 450, revêtues d'antiimmunoglobuline humaine en appliquant la méthode décrite dans l'exemple 1 de la demande de brevet WO 90/09590 et on a introduit 25 µl de ces particules dans les cupules contenant les dilutions d'anti-D. On a alors traité la plaque comme décrit dans l'exemple 1 de ladite demande (homogénéisation sur microagitateur puis action d'un champ magnétique statique). La dernière dilution donnant une distribution positive difficilement observable à l'oeil nu est celle au 1/16^{e} alors qu'avec les mêmes particules soumises au traitement magnétique décrit dans le présent exemple, une distribution nette est observable jusqu'au 1/32^{e} avec une discrimination parfaite rendant l'interprétation très aisée.

Enfin, on a utilisé les particules magnétiques Estapor^{R} sensibilisées, de 0,8 µm (µ), et on les a soumises au traitement magnétique décrit dans WO 90/09590; les figures obtenues sont ininterprétables puisque le témoin négatif lui-même ne donne pas le point attendu en fond de cupule, même si la durée d'action du champ magnétique est prolongée jusqu'à 30 minutes alors que les mêmes particules soumises au traitement magnétique de l'invention donnent une discrimination parfaite au 1/128^{e}.

### EXEMPLE 2

Les microplaques sont préparées comme à l'exemple 1 mais le traitement magnétique est effectué en trois temps, les deux premiers temps étant identiques à ceux de l'exemple précédent; pendant le troisième, on introduit durant 2 minutes des tiges aimantées, telles que précédemment décrites, de diamètre 3 mm dans chaque cupule. Après retrait des tiges, on distingue alors à la loupe binoculaire une distribution positive jusqu'à une dilution au 1/1024^{e} de la dilution mère à 1 %.

### EXEMPLE 3

Le traitement magnétique des plaques préparées comme à l'exemple 1 est modifié pour ne plus comporter que le premier et le troisième temps du traitement décrit à l'exemple 2.

L'observation avec un photomètre convenable permet de constater la présence d'une distribution positive jusqu'à une dilution au 1/256^{e} de la dilution mère à 1 %.

### EXEMPLE 4 Groupage sanguin ABO, épreuve dite de Simonin.

On prépare une suspension à 0,02 % (p/V) de particules magnétiques Estapor^{R} 0,8 µm revêtues d'antiglobulines humaines, ainsi que des plaques de microtitration revêtues d'hématies de capture comme à l'exemple 1 mais de groupe A1, A2 et B. 50 µl de plasma à étudier et 100 µl de solution de basse force ionique sont introduits dans chaque cupule et on fait incuber pendant 20 minutes à 37° C. Les cupules sont ensuite lavées et 50 µl de suspension de particules magnétiques y sont introduites, avant de soumettre la plaque à un traitement magnétique en deux temps comme à l'exemple 2. L'observation des distributions obtenues a permis de déterminer, sans erreur, le groupe A, B, AB ou O du plasma étudié.

### EXEMPLE 5 Groupage sanguin ABO, épreuve globulaire dite de Beth Vincent.

Dans cet essai, on utilise une plaque de microtitration en polystyrène dont les cupules en forme de U sont prétraitées pour qu'elles puissent fixer les hématies, par la méthode décrite dans Methods in enzymology (Vol. 73 - C.H. Heusser; J.W. Stocker, R.H. Gisler Academic Press Inc. - 1981).

On prépare, comme à l'exemple 1, une suspension à 0,02 % (p/v) de particules magnétiques Estapor^{R}, de 0,8 µm de diamètre, mais revêtues d'antiglobulines murines.

50 µl d'une suspension à 1 % (p/v) des hématies à étudier, préalablement lavées, sont distribués dans 3 cupules par échantillon et la plaque est laissée 5 minutes à température ambiante avant le lavage des cupules avec du sérum physiologique. Dans l'une des 3 cupules d'un échantillon, on introduit 50 µl de sérum-test murin anti-A avec 100 µl de solution de basse force ionique, dans une autre 50 µl de sérum-test murin anti-B avec la même quantité de solution de basse force ionique, et dans la troisième cupule du sérum-test anti-A+B ainsi que de la solution de basse force ionique. Après 20 minutes d'incubation à 37° C, les cupules sont lavées et on introduit dans chacune 50 µl de suspension de particules magnétiques, avant de soumettre la plaque à un traitement magnétique en trois temps comme à l'exemple 2.

L'observation de la répartition des particules magnétiques a permis de déterminer sans erreur le groupe A, B, AB ou O de chacun des 70 échantillons dont certains présentaient une faible antigénicité, étant des groupes A₃, A₃B, Ax, B₃ ou Bh.

### EXEMPLE 6 Dosage d'immunoglobulines de type Ig G.

On prépare une suspension de particules magnétiques sensibilisées avec un anticorps anti-Ig G humaines comme à l'exemple 1.

Par ailleurs, on revêt les 96 cupules d'une plaque de microtitration en polystyrène rigide, commercialisée par la société Polylabo (France) sous la référence M 24 A, d'un anticorps de chèvre anti-Ig G humaines comme suit : on introduit 100 µl d'une solution aqueuse à 2,2 mg/ml d'anti-IgG et laisse incuber pendant 1 heure à 37° C et une nuit à 4° C. Après élimination du liquide, on remplit les godets de tampon GBS contenant 0,1 % de sérum albumine bovine, et on laisse au contact pendant une nuit à 4° C, puis on lave abondamment avec une solution aqueuse de NaCl à 0,9 % (p/V). Les échantillons à étudier, de concentration 1 µg/ml, sont préparés en dissolvant l'immunoglobuline IgG commercialisée par Sigma sous la référence I 4506 dans une solution aqueuse de NaCl (0,9 %) contenant 2 % de sérum albumine; les échantillons témoins négatifs ne comportent que le diluant. On introduit dans chaque cupule de la plaque, 100 µl d'échantillon et laisse incuber pendant 45 minutes à 37° C avant de laver par une solution saline.

On introduit alors dans chaque cupule 50 µl de la suspension de particules magnétiques amenée à la concentration de 0,02 % en tampon GBS - pH 8,2, additionné de 0,1 % de sérum albumine bovine, et on soumet la plaque au traitement magnétique décrit à l'exemple 2. On différencie parfaitement à l'oeil nu les cupules positives des négatives.

### EXEMPLE 7 Dépistage d'anticorps antiérythrocytaires irréguliers.

Les solutions de particules magnétiques et celles des sérums ou plasmas à étudier contenant des anticorps irréguliers (dilutions jusqu'au 1/2048^{e}) sont préparés comme à l'exemple 1.

On étudie d'une part 26 sérums ou plasmas contenant des alloanticorps irréguliers de spécificité C, c, E, D, K, Kpb, Fya, Fyb, Jka, Jkb, S ou Lub et, d'autre part, 170 plasmas dits négatifs que l'on sait ne contenir aucun anticorps irrégulier.

Les cupules sont prétraitées comme à l'exemple 5, puis 50 µl d'une suspension à 1 % (p/v) des hématies-test, dont la spécificité vis-à-vis de l'anticorps à rechercher est connue, est introduite dans les cupules. Celles-ci sont lavées après 5 minutes de repos à la température ambiante.

Pour l'étude des plasmas contenant les anticorps irréguliers, on prépare pour chaque échantillon une cupule avec hématies-test portant l'antigène correspondant à l'anticorps recherché et une cupule avec hématies-test ne comportant pas cet antigène.

Pour l'étude des plasmas négatifs, non dilués, une cupule contient des hématies-test portant l'ensemble des antigènes de groupe sanguin.

Dans les cupules lavées, on introduit 50 µl de la solution à étudier et 100 µl de solution aqueuse de basse force ionique et on laisse incuber 20 minutes à 37° C. Les cupules sont ensuite lavées avant d'y introduire 50 µl de la suspension de particules magnétiques et de soumettre la plaque au traitement en trois temps décrit à l'exemple 2, avant d'observer la répartition des particules soit à l'oeil soit au photomètre.

Aucune réaction faussement positive n'a été décelée sur les plasmas négatifs, ce qui montre la bonne spécificité du procédé.

Les titres, c'est-à-dire la dernière dilution pour laquelle on a observé une réaction positive, pour les échantillons d'anticorps étudiés, figurent dans le Tableau I.

On a répété 10 fois, en plusieurs occasions, la détermination du titre sur un sérum contenant l'anticorps anti-D; aucune différence significative du titre au 1/128^{e} n'a été observée.

A titre de comparaison, on a effectué le dosage des 26 échantillons d'anticorps irréguliers avec l'ensemble commercialisé sous la marque Capture R d'Immucor. On a constaté que le procédé selon l'invention donnait, pour 56 % des échantillons, un gain de sensibilité net correspondant à une différence de titre de 1 à 3.

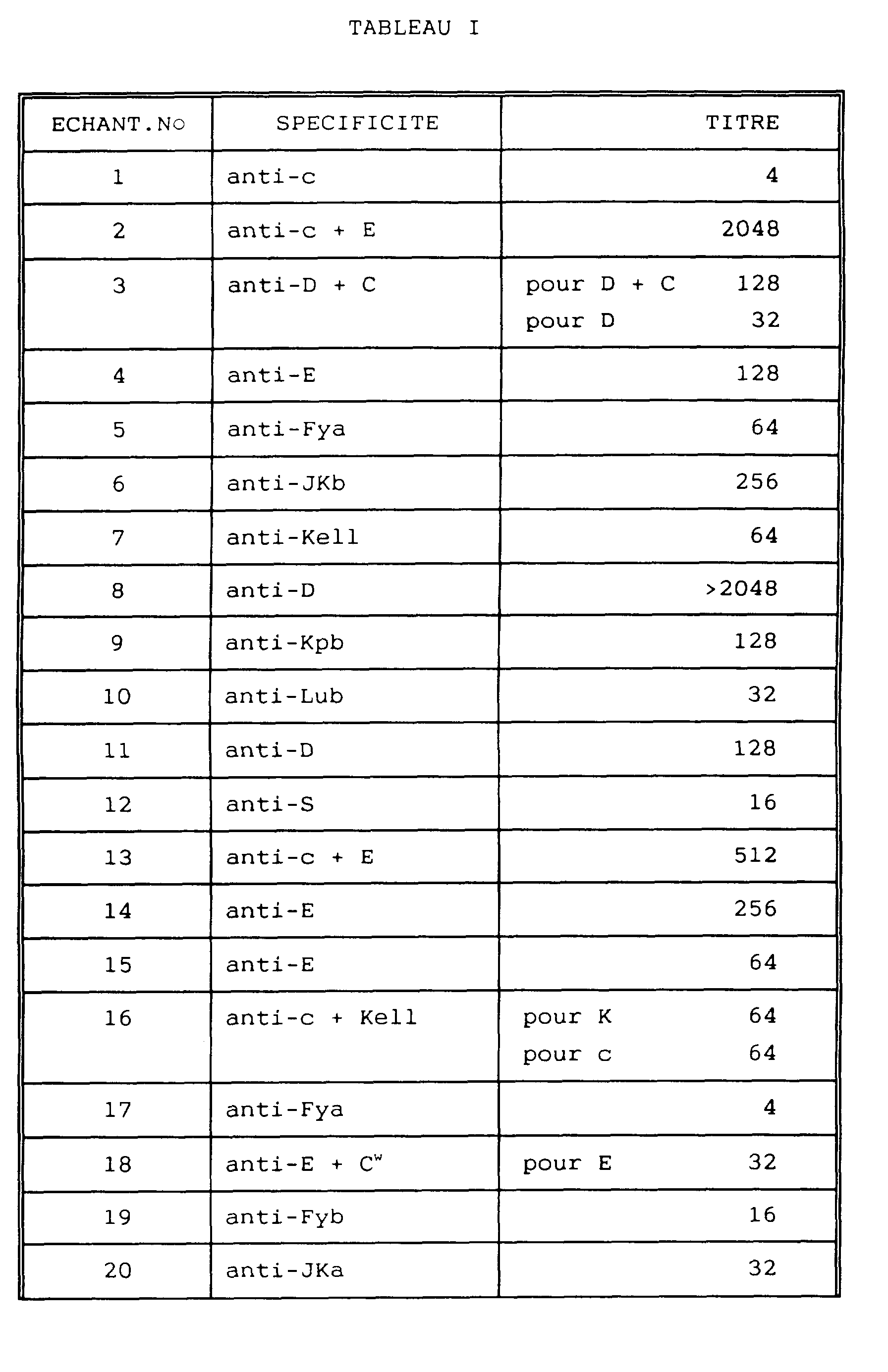

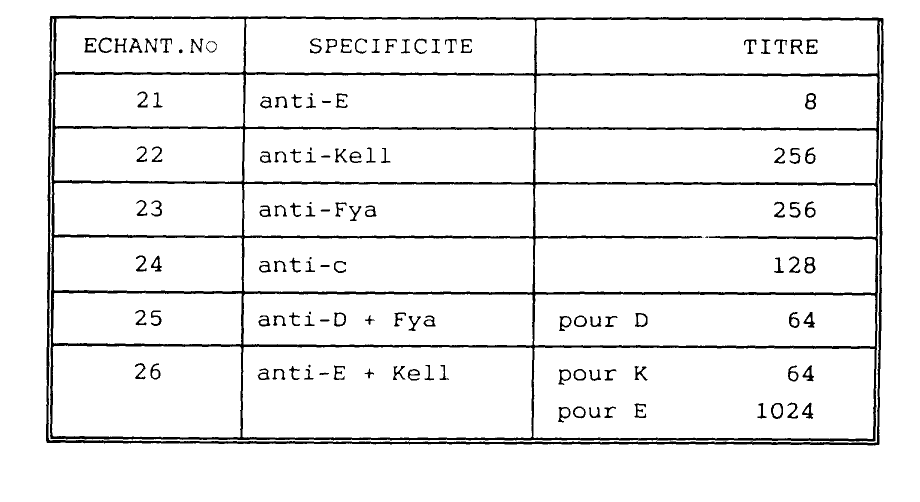

## Revendications

1. Procédé de dosage ou de détection par immunoadhérence d'une substance biologique susceptible d'être présente dans un échantillon, qui consiste à introduire l'échantillon dans un récipient dont les parois portent un élément ayant une affinité immunologique spécifique pour la substance à rechercher, à ajouter des particules magnétiques portant une substance ayant une affinité immunologique spécifique pour la substance à rechercher, à les soumettre à plusieurs actions magnétiques successives pour accélérer le dépôt desdites particules sur les parois et déplacer celles qui ne sont pas entrées dans des liaisons spécifiques avec la substance à rechercher qui adhère aux parois par l'intermédiaire de l'élément affine qui y est fixé, et à observer les particules déposées.

2. Procédé selon la revendication 1 qui consiste :
a) à introduire ledit échantillon dans un récipient qui porte sur ses parois un élément ayant une affinité immunologique spécifique pour la substance à rechercher,
b) à ajouter des particules magnétiques portant une substance ayant une affinité immunologique spécifique pour la substance à rechercher,
c) à soumettre les particules à l'action d'un champ magnétique statique pour plaquer les particules sur les parois, et permettre ainsi la réaction immunologique,
d) puis à soumettre les particules à l'action d'un champ magnétique d'orientation variable pour arracher et réunir au fond les particules qui ne sont pas fixées par liaison spécifique aux parois par l'intermédiaire de la substance à rechercher et de l'élément affine solidaire de la paroi,
et enfin,
e) à observer les particules déposées.

3. Procédé selon la revendication 1 qui consiste :
a) à introduire ledit échantillon dans un récipient qui porte sur ses parois un élément ayant une affinité immunologique spécifique pour la substance à rechercher,
b) à ajouter des particules magnétiques portant une substance ayant une affinité immunologique spécifique pour la substance à rechercher,
c) à soumettre les particules à l'action d'un champ magnétique statique pour les plaquer sur les parois et permettre ainsi la réaction immunologique,
d) puis à soumettre les particules à l'action d'un champ magnétique d'orientation variable pour arracher et réunir au fond celles qui ne sont pas fixées par liaison spécifique aux parois par l'intermédiaire de la substance à rechercher et de l'élément affine solidaire de la paroi,
e) à soumettre les particules à l'action d'un champ magnétique statique pour retirer du récipient celles qui n'y adhèrent pas par l'intermédiaire de liaisons immunologiques,
f) et à observer les particules déposées.

4. Procédé selon la revendication 1 qui consiste :
a) à introduire ledit échantillon dans un récipient qui porte sur ses parois un élément ayant une affinité immunologique spécifique pour la substance à rechercher,
b) à ajouter des particules magnétiques portant une substance ayant une affinité immunologique pour ladite substance à rechercher,
c) à soumettre les particules à l'action d'un champ magnétique statique pour les plaquer sur les parois et permettre ainsi la réaction immunologique caractéristique,
d) puis à soumettre le récipient à l'action d'un champ magnétique statique pour retirer du récipient celles qui n'adhèrent pas à ses parois par une liaison de type immunologique,
e) et à observer les particules déposées.

5. Procédé selon l'une des revendications 1 à 4, caractérisé en ce qu'on effectue, en outre, une incubation puis un lavage entre les étapes a et b.

6. Procédé selon l'une des revendications 1 à 3, caractérisé en ce que le champ d'orientation variable résulte d'un déplacement rotatif du récipient dans un champ magnétique statique.

7. Procédé selon l'une des revendications 3 et 4, caractérisé en ce que pour retirer les particules qui n'adhèrent pas, on introduit dans le récipient une tige convenablement aimantée sur laquelle uniquement ces particules viennent se fixer.

8. Procédé selon l'une des revendications 1 à 7, caractérisé en ce qu'il est effectué simultanément dans plusieurs cupules d'une plaque de microtitration.

9. Procédé selon la revendication 8, caractérisé en ce que les particules magnétiques ont des diamètres compris entre 0,5 µm et 5 µm.

10. Procédé selon la revendication 9, caractérisé en ce que les particules magnétiques ont des diamètres de 0,8 à 1,7 µm.

11. Dispositif pour la mise en oeuvre, dans une plaque de microtitration, d'un procédé selon la revendication 2 qui comporte :
- un support (3) maintenant à l'horizontale une plaque de microtitration (1) susceptible de donner à la plaque un mouvement de translation circulaire horizontal,
- un ensemble (5) d'aimants (6) dont les pôles supérieurs sont parallèles à la plaque (1) et sont chacun sous une cupule (2),
les aimants étant soit disposés en damier de pôles alternativement Nord-Sud, soit disposés en bandes de pôles alternés.

12. Dispositif pour la mise en oeuvre, dans une plaque de microtitration, d'un procédé selon la revendication 2, qui comporte :
- un support fixe maintenant à l'horizontale une plaque de microtitration,
- un ensemble d'aimants dont les pôles supérieurs sont parallèles à la plaque et sont chacun sous une cupule, les aimants étant soit disposés en damier de pôles alternativement Nord- Sud, soit disposés en bandes de pôles alternés, cet ensemble étant muni de moyens pour lui donner un mouvement de translation circulaire horizontal.

13. Dispositif selon l'une des revendications 11 et 12, caractérisé en ce que l'ensemble d'aimants est constitué de bandes ou de carrés d'un polymère aimanté.

14. Dispositif, pour la mise en oeuvre dans une plaque de microtitration, d'un procédé selon la revendication 3 ou la revendication 4, qui comporte une série de tiges aimantées (22) et des moyens pour les introduire simultanément dans toutes les cupules (2) et les en extraire.

15. Appareil d'analyse biologique comprenant un dispositif selon l'une quelconque des revendications 11 à 13 et/ou selon la revendication 14.

## Patentansprüche

1. Verfahren zur Dosierung oder zum Nachweis einer biologischen Substanz, die in einer Probe vorhanden sein kann, durch Immunadhäsion, bestehend aus Eingeben der Probe in einen Behälter, dessen Wände ein Element tragen, das eine spezifische immunologische Affinität zu der zu suchenden Substanz aufweist, Zugeben von magnetischen Partikeln, die eine Substanz mit einer spezifischen immunologischen Affinität zu der zu suchenden Substanz tragen, Ausüben von mehreren aufeinanderfolgenden magnetischen Wirkungen auf die Partikel, um die Ablagerung der genannten Partikel an den Wänden zu beschleunigen und diejenigen zu entfernern, die keine spezifischen Bindungen mit der an den Wänden haftenden zu suchenden Substanz, vermittelt durch das an ihr befestigte affine Element, eingegangen sind, und Beobachten der abgelagerten Partikel.

2. Verfähren nach Anspruch 1, bestehend aus:
a) Eingeben der genannten Probe in einen Behälter, der an seinen Wänden ein Element trägt, das eine spezifische immunologische Affinität zu der zu suchenden Substanz aufweist,
b) Zugeben von magnetischen Partikeln, die eine Substanz mit einer spezifischen immunologischen Affinität zu der zu suchenden Substanz tragen,
c) Einwrkenlassen eines statischen Magnetfelds auf die Partikel, um die Partikel an den Wänden abzuscheiden und so die inmunologische Reaktion zu ermöglichen,
d) anschließendes Einwirkenlassen eines Magnetfelds mit veränderlicher Richtung auf die Partikel, um diejenigen Partikel, die nicht durch spezifische Bindung mit Hilfe der zu suchenden Substanz und dem an der Wand sitzenden affinen Element an den Wänden befestigt worden sind, abzulösen und am Boden zu sammeln, und schließlich
e) Beobachten der abgelagerten Partikel.

3. Verfahren nach Anspruch 1, bestehend aus:
a) Eingeben der genannten Probe in einen Behälter, der an seinen Wänden ein Element trägt, das eine spezifische immunologische Affinität zu der zu suchenden Substanz aufweist,
b) Zugeben von magnetischen Partikeln, die eine Substanz mit einer spezifischen immunologischen Affinität zu der zu suchenden Substanz tragen,
c) Einwirkenlassen eines statischen Magnetfelds auf die Partikel, um sie an den Wänden abzuscheiden und so die immunologische Reaktion zu ermöglichen,
d) anschließendes Einwirkenlassen eines Magnetfelds mit variabler Richtung auf die Partikel, um diejenigen, die nicht durch spezifische Bindung mit Hilfe der zu suchenden Substanz und des an der Wand sitzenden affinen Elements an den Wänden befestigt worden sind, abzulösen und am Boden zu sammeln,
e) Einwirkenlassen eines statischen Magnetfelds auf die Partikel, um diejenigen aus dem Behälter abzuziehen, die nicht vermittels immunologischer Bindungen daran haften, und
f) Beobachten der abgelagerten Partikel.

4. Verfahren nach Anspruch 1, bestehend aus:
a) Eingeben der genannten Probe in einen Behälter, der an seinen Wänden ein Element trägt, das eine spezifische immunologische Affinität zu der zu suchenden Substanz aufweist,
b) Zugeben von magnetischen Partikeln, die eine Substanz mit einer immunologischen Affinität zu der zu suchenden Substanz tragen,
c) Einwirkenlassen eines statischen Magnetfelds auf die Partikel, um sie an den Wänden abzuscheiden und so die charakteristische immmunologische Reaktion zu ermöglichen,
d) anschließendes Einwirkenlassen eines statischen Magnetfelds auf den Behälter, um diejenigen aus dem Behälter abzuziehen, die nicht durch eine Bindung immunologischer Art an seinen Wänden haften, und
e) Beobachten der abgelagerten Partikel.

5. Verfahren nach einem der Ansprüche 1 bis 4, dadurch gekennzeichnet, daß man zwischen den Schritten a und b außerdem eine Inkubation und ein anschließendes Waschen vornimmt.

6. Verfahren nach einem der Ansprüche 1 bis 3, dadurch gekennzeichnet, daß das Feld mit veränderlicher Richtung aus einer Drehbewegung des Behälters in einem statischen Magnetfeld resultiert.

7. Verfahren nach einem der Ansprüche 3 und 4, dadurch gekennzeichnet, daß man zum Abziehen der nicht haftenden Partikel einen geeignet magnetisierten Stab in den Behälter einführt, an dem sich ausschließlich diese Partikel festsetzen.

8. Verfahren nach einem der Ansprüche 1 bis 7, dadurch gekennzeichnet, daß es gleichzeitig in mehreren Näpfchen einer Mikrotitrationsplatte stattfindet.

9. Verfahren nach Anspruch 8, dadurch gekennzeichnet, daß die magnetischen Partikel Durchmesser zwischen 0,5 µm und 5 µm haben.

10. Verfahren nach Anspruch 9, dadurch gekennzeichnet, daß die magnetischen Partikel Durchmesser zwischen 0,8 und 1,7 µm haben.

11. Vorrichtung zur Durchführung eines Verfahrens nach Anspruch 2 in einer Mikrotitrationsplatte, mit:
- einem Träger (3), der eine Mikrotitrationsplatte (1) waagerecht hält und in der Lage ist, ihr eine waagerechte, kreisförmige Translationsbewegung zu erteilen,
- einer Gruppe (5) von Magneten (6), deren obere Pole zu der Platte (1) parallel sind und jeweils unter einem Näpfchen (2) liegen,
wobei die Magnete entweder schachbrettartig mit abwechselnden Nord- und Südpolen oder in Streifen mit abwechselnden Polen angeordnet sind.

12. Vorrichtung zur Durchführung eines Verfahrens nach Anspruch 2 in einer Mikrotitrationsplatte, mit:
- einem festen Träger, der eine Mikrotitrationsplatte waagerecht hält,
- einer Gruppe von Magneten, deren obere Pole zu der Platte parallel sind und jeweils unter einem Näpfchen liegen, wobei die Magnete entweder schachbrettartig mit abwechselnden Nord- und Südpolen oder in Streifen mit abwechselnden Polen angeordnet sind und diese Gruppe mit Mitteln versehen ist, die ihr eine waagerechte kreisförmige Translationsbewegung erteilen.

13. Vorrichtung nach einem der Ansprüche 11 und 12, dadurch gekennzeichnet, daß die Magnetgruppe durch Streifen oder Quadrate eines magnetischen Polymers gebildet sind.

14. Vorrichtung zur Durchführung eines Verfahrens nach Anspruch 3 oder 4 in einer Mikrotitrationsplatte, mit einer Folge von magnetischen Stäben (22) und Mitteln zum simultanen Einführen derselben in und zum Herausziehen derselben aus sämtlichen Näpfchen (2).

15. Biologisches Analysegerät mit einer Vorrichtung nach einem der Ansprüche 11 bis 13 und/oder nach Anspruch 14.

## Claims

1. Process for measuring or detecting by immunoadherence a biological substance which may be present in a sample, which consists in placing the sample in a container the walls of which are provided with an element having a specific immunological affinity for the substance under investigation, adding magnetic particles carrying a substance having a specific immunological affinity for the substance under investigation, subjecting them to a number of successive magnetic actions in order to accelerate the depositing of said particles on the walls and displace those which have not entered into specific bonds with the substance under investigation, which adheres to the walls by means of the element with affinity which is fixed thereon, and observing the particles deposited.

2. Process according to claim 1 which consists in:
a) placing said sample in a container which has, on its walls, an element having a specific immunological affinity for the substance under investigation,
b) adding magnetic particles carrying a substance having a specific immunological affinity for the substance under investigation,
c) subjecting the particles to the action of a static magnetic field in order to apply the particles to the walls and thus enable the immunological reaction to take place,
d) then subjecting the particles to the action of a magnetic field of variable orientation in order to pull off and re-settle those particles which are not fixed to the walls by specific binding by means of the substance under investigation and the element with affinity which is attached to the wall,
and finally
e) observing the particles deposited.

3. Process according to claim 1 which consists in:
a) placing said sample in a container which has on its walls an element having a specific immunological affinity for the substance under investigation,
b) adding magnetic particles carrying a substance having a specific immunological affinity for the substance under investigation,
c) subjecting the particles to the action of a static magnetic field in order to apply them to the walls and thus enable the immunological reaction to take place,
d) then subjecting the particles to the action of a magnetic field of variable orientation in order to pull off and re-settle those particles which are not fixed to the walls by specific binding by means of the substance under investigation and the element with affinity which is attached to the wall,
e) subjecting the particles to the action of a static magnetic field in order to remove from the container those particles which are not adhering thereto by means of immunological bonds,
f) and observing the particles deposited.

4. Process according to claim 1, which consists in:
a) placing said sample in a container which has on its walls an element having a specific immunological affinity for the substance under investigation,
b) adding magnetic particles carrying a substance having an immunological affinity for said substance under investigation,
c) subjecting the particles to the action of a static magnetic field in order to apply them to the walls and thus allow the characteristic immunological reaction to take place,
d) then subjecting the container to the action of a static magnetic field in order to remove from the container those particles which are not adhering to its walls by means of an immunological bond,
e) and observing the particles deposited.

5. Process according to one of claims 1 to 4, characterised in that incubation followed by washing is also carried out, between steps a and b.

6. Process according to one of claims 1 to 3, characterised in that the field of variable orientation results from rotary movement of the container in a static magnetic field.

7. Process according to one of claims 3 and 4, characterised in that, in order to remove the non-adhering particles, a suitably magnetised rod to which only these particles will adhere is placed in the container.

8. Process according to one of claims 1 to 7, characterised in that it is carried out simultaneously in several wells of a microtitre plate.

9. Process according to claim 8, characterised in that the magnetic particles have diameters of from 0.5 to 5 µm.

10. Process according to claim 9, characterised in that the magnetic particles have diameters of from 0.8 to 1.7µm.

11. Apparatus for carrying out a process according to claim 2 in a microtitre plate, said apparatus comprising:
a support (3) which holds a microtitre plate (1) horizontal and which is capable of imparting a circular horizontal movement of translation to the plate,
a set (5) of magnets (6) the upper poles of which are parallel to the plate (1) and are each located beneath a well (2),
the magnets being arranged either in a chequerboard arrangement of alternate north-south poles or in rows of alternate poles.

12. Apparatus for carrying out a process according to claim 2 in a microtitre plate, said apparatus comprising:
a fixed support which holds a microtitre plate in a horizontal position,
a set of magnets the upper poles of which are parallel to the plate and are each located beneath a well, the magnets being arranged either in a chequerboard arrangement of alternate north-south poles or in rows of alternate poles, this set being provided with means of imparting a circular, horizontal movement of translation thereto.

13. Apparatus according to one of claims 11 and 12, characterised in that the set of magnets consists of strips or squares of a magnetised polymer.

14. Apparatus for carrying out a process according to claim 3 or 4 in a microtitre plate, said apparatus comprising a series of magnetised rods (22) and means for introducing them simultaneously into all the wells (2) and removing them therefrom.

15. Apparatus for biological analysis, comprising an apparatus according to any one of claims 11 to 13 and/or according to claim 14.
